Europäisches Patentamt

European Patent Office

Office européen des brevets

(19)

(11) Numéro de publication : **0 485 279 A1**

## (12) DEMANDE DE BREVET EUROPEEN

(21) Numéro de dépôt : **91402966.5**

(22) Date de dépôt : **06.11.91**

(51) Int. Cl.⁵ : **A61B 17/32**

(30) Priorité : **06.11.90 FR 9013710**

(43) Date de publication de la demande :
**13.05.92 Bulletin 92/20**

(84) Etats contractants désignés :
**AT BE CH DE DK ES GB GR IT LI LU NL**

(71) Demandeur : **ETHICON INC.**
**U.S. Route 22**
**Somerville New Jersey 08876 (US)**

(72) Inventeur : **Bilweis, Joseph**
**10, l'Orée de Marly**
**F-78560 Noisy le Roi (FR)**

(74) Mandataire : **Martin, Jean-Jacques et al**
**Cabinet REGIMBEAU 26, Avenue Kléber**
**F-75116 Paris (FR)**

(54) **Dissecteur, notamment pour chirurgie endoscopique.**

(57)    La présente concerne un dissecteur, notamment pour chirurgie endoscopique, caractérisé par le fait qu'il comprend : une platine support (110), un élément de commande (120) susceptible de translation par rapport à la platine support (110) et pourvu d'un doigt de commande (115) transversal à sa direction de translation, et deux lames (160, 170) portées par la platine (110), dont l'une au moins est articulée sur la platine (110) et possède une rampe (162, 172) inclinée par rapport à la direction de translation de l'élément de commande (120) et sur laquelle repose le doigt de commande (125), de sorte que le déplacement à translation de l'élément de commande (120), par rapport à la platine support (110), entraîne un pivotement relatif des deux lames (160, 170).

FIG.4

EP 0 485 279 A1

La présente invention concerne le domaine des instruments chirurgicaux.

La présente invention concerne plus précisément un dissecteur, notamment pour chirurgie endoscopique.

On sait que des dissecteurs doivent souvent être utilisés en chirurgie. Les dissecteurs sont des instruments chirurgicaux destinés à cliver ou séparer des tissus non étroitement liés. Les dissecteurs généralement utilisés sont formés de spatules à bords mousses, c'est-à-dire arrondis.

Cependant les dissecteurs jusqu'ici proposés ne donnent pas totalement satisfaction.

En particulier, à la connaissance de la Demanderesse, les dissecteurs jusqu'ici proposés ne peuvent être utilisés lors d'interventions endoscopiques.

La présente invention a pour but principal d'améliorer les dissecteurs existants, en particulier en proposant un nouveau dissecteur apte à être utilisé en chirurgie endoscopique.

Ce but est atteint selon la présente invention, grâce à un dissecteur comprenant :

– une canule,

– une platine support placée dans la canule et formée d'une tringle allongée de longueur supérieure à celle de la canule,

– un élément de commande susceptible de translation par rapport à la platine support et pourvu d'un doigt de commande transversal à la direction de translation, et

– deux lames, portées par la platine, dont l'une au moins est articulée sur ladite platine et possède une rampe inclinée par rapport à la direction de translation de l'élément de commande, et sur laquelle repose le doigt de commande, de sorte que le déplacement à translation de l'élément de commande, par rapport à la platine support, entraîne un pivotement relatif des deux lames, dont le bord extérieur non tranchant est adapté au clivage.

D'autres caractéristiques, buts et avantages de la présente invention apparaîtront à la lecture de la description détaillée qui va suivre et en regard des dessins annexés, donnés à titre d'exemples non limitatifs et sur lesquels :

– la figure 1 représente une première vue latérale d'un dissecteur conforme à un premier mode de réalisation de la présente invention, placé dans une canule représentée en vue axiale longitudinale,

– la figure 2 représente une seconde vue latérale, orthogonale à la figure 1, du même dissecteur placé dans une canule, en position fermée,

– la figure 3 représente une vue similaire à la figure 1 du dissecteur sortie de la canule,

– la figure 4 représente une vue similaire à la figure 2 du même dissecteur en position ouverte des lames,

– les figures 5A et 5B représentent des vues schématiques en plan de deux lames séparées d'un dissecteur conforme à un second mode de réalisation de la présente invention,

– la figure 6 représente une vue similaire aux figures 1 et 3 du dissecteur assemblé conforme au second mode de réalisation,

– la figure 7 représente une vue similaire aux figures 2 et 4 du dissecteur conforme au second mode de réalisation, en position ouverte des lames,

– les figures 8A et 8B représentent deux vues d'un dissecteur conforme à un troisième mode de réalisation de la présente invention, respectivement en position fermée et en position ouverte des lames,

– les figures 9A et 9B représentent des vues d'un dissecteur conforme à un quatrième mode de réalisation de la présente invention, respectivement en position fermée et en position ouverte des lames,

– les figures 10A et 10B représentent deux vues en plan de deux lames séparées d'un dissecteur conforme à un cinquième mode de réalisation de la présente invention, tandis que

– la figure 10C représente une vue du dissecteur conforme à ce cinquième mode de réalisation, en position ouverte des lames.

Les représentations données sur les figures annexées ne sont bien entendu que schématiques et ne doivent aucunement être considérées comme limitatives, en particulier en ce qui concerne les dimensions relatives des différents éléments composants le dissecteur.

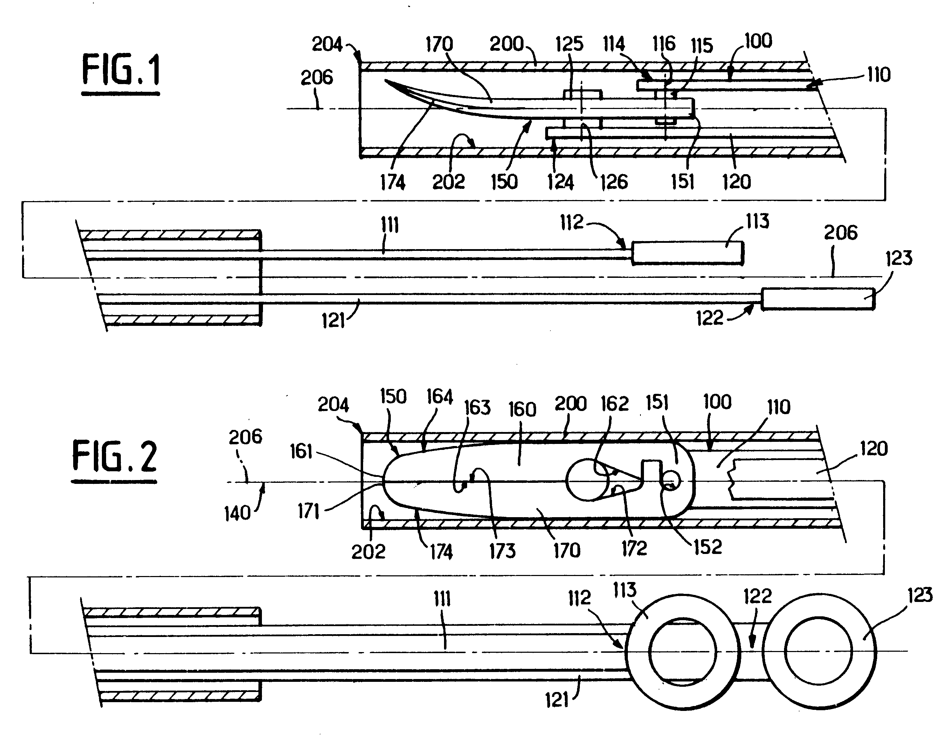

On aperçoit sur les figures 1 à 4 annexées un dissecteur 100 placé dans une canule 200.

Le dissecteur 100 comprend essentiellement une platine 110, deux lames 160, 170 et un élément de commande 120.

En position fermée des lames 160, 170, comme représentées sur les figures 1 et 2, l'encombrement transversal du dissecteur est inférieur à la section libre de la lumière 202 de la canule 200 de sorte que la partie active du dissecteur formée par les lames 160, 170, puisse être logée entièrement dans la canule 200.

On voit nettement sur les figures 1 et 2 que les extrémités 161, 171 des lames 160, 170 sont placées en retrait de l'extrémité proximale 204 de la canule.

Par ailleurs, en position fermée des lames, l'ensemble du dissecteur 100 peut être déplacé à translation dans la canule 200, selon l'axe 206 de celle-ci.

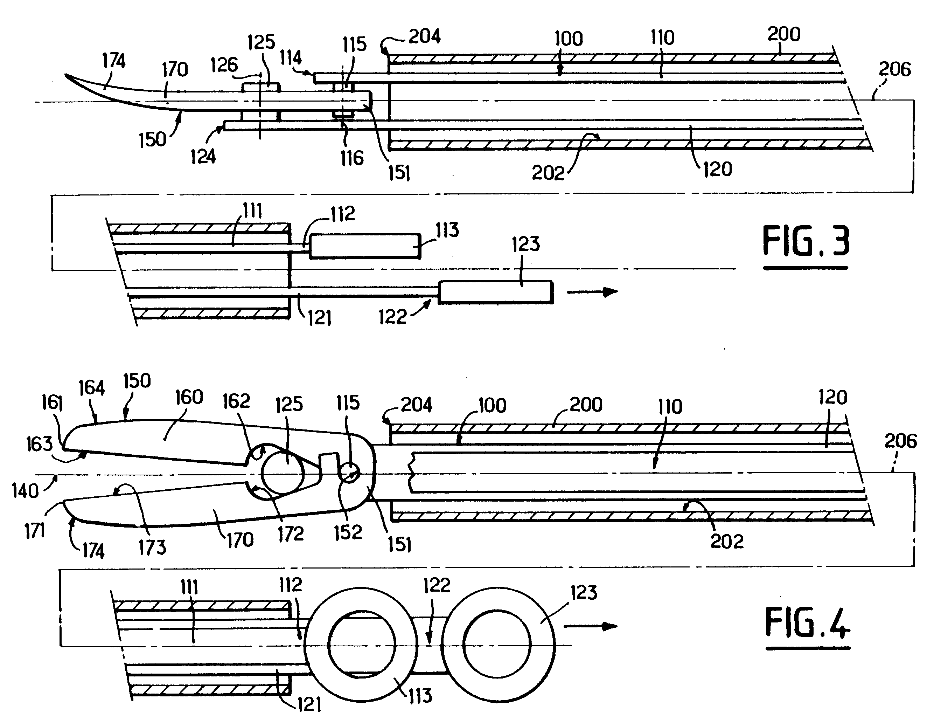

Lorsque comme représenté sur les figures 3 et 4, les lames 160, 170, sont ainsi portées à l'extérieur de l'extrémité proximale 204 de la canule 200, les lames 160, 170, peuvent être déplacées en position d'ouverture comme illustré sur la figure 4, par actionnement de l'élément commande 120.

Selon la représentation schématique donnée sur les figures 1 à 4 annexées, la platine 110 est formée d'une tringle allongée 111. La tringle 111 possède une longueur supérieure à la longueur de la canule 200. La tringle 111 est munie à son extrémité distale 112 de moyens de préhension 113, tels que par exemple comme représenté sur les figures annexées d'un oeillet 113 conçu pour être saisi par un doigt d'un opérateur.

Par ailleurs, la tringle 111 est munie à son extrémité proximale 114 d'un tourillon 115 centré sur un axe 116 perpendiculaire à l'axe 206 de la canule 200. Les lames 160, 170, sont conçues pour être susceptibles de pivotement relatif autour de cet axe 116 formé par le tourillon 115.

Selon le mode de réalisation représenté sur les figures 1 à 4 annexées, les deux lames 160, 170 sont formées d'une pièce unique 150 repliée sur elle-même en forme générale de U.

L'âme 151 de la pièce 150 définit un palier cylindrique 152 par lequel la pièce 150 formant les lames 160, 170 est engagée sur le tourillon 115 pour être guidée à pivotement autour de l'axe 116.

La pièce 150, et par conséquent les lames 160, 170 sont de préférence symétriques par rapport à un plan axial 140. Ce plan de symétrie 140 coïncide avec l'axe de pivotement 116. Le plan de symétrie 140 est parallèle au plan des figures 1 et 3, et est perpendiculaire au plan des figures 2 et 4.

Les lames 160, 170 sont munies chacune d'une rampe 162, 172 inclinée par rapport au plan de symétrie 140 précité. Les rampes 162, 172, sont également inclinées par rapport à la direction de translation de l'élément de commande 120 en regard de la platine 110, puisque comme cela sera précisé par la suite, la direction de translation de l'élément de commande 120 coïncide avec le plan de symétrie 140 précité.

Les rampes 162, 172 sont de préférence définies par une génératrice parallèle à l'axe 116 et parallèle au plan de symétrie 140.

Selon le mode de réalisation représenté sur les figures annexées, les rampes 162, 172 sont définies sur les bords internes en regard 163, 173 des lames 160, 170, c'est-à-dire les bords de ces lames dirigés vers le plan de symétrie 140.

L'élément de commande 120 est formé d'une tige 121 parallèle à la tringle 110. La tige 121 est susceptible de translation par rapport à la tringle 111 formant la platine, parallèlement au plan de symétrie 140 et à l'axe 206 de la canule.

La tige 121 est munie à son extrémité distale 122 de moyens de préhension, tels que par exemple un oeillet 123 similaire à l'oeillet 113 précité.

A son extrémité proximale 124, la tige 120 est munie d'un doigt de commande 125 qui lui est transversal.

Selon le mode de réalisation particulier représenté sur les figures 1 à 4 annexées, le doigt 125 est un doigt cylindrique centré sur un axe 126 qui est orthogonal à l'axe 206 de la canule et qui coïncide avec le plan de symétrie 140.

Le doigt 125 est engagé entre les rampes 162, 172. Plus précisément encore, selon le mode de réalisation représenté sur les figures 1 à 4, les rampes 162, 172 sont formées sur le côté proximal du tourillon d'articulation 115. Par ailleurs, les rampes 162, 172 convergent en rapprochement de ce tourillon 115.

En outre, les lames 160, 170 représentées sur les figures 1 à 4 sont sollicitées à la fermeture. De préférence les lames 160, 170 sont sollicitées vers leur position de fermeture représentée sur la figure 2 grâce à l'élasticité inhérente à la pièce 150 composant les deux lames 160, 170. En variante cependant les lames 160, 170 peuvent être sollicitées vers la position de repos fermée représentée sur la figure 2 grâce à un ressort additionnel. Un tel ressort additionnel peut faire l'objet de nombreux modes de réalisation. Il peut s'agir par exemple d'un ressort hélicoïdal engagé sur le tourillon 115 et dont les extrémités viennent respectivement en prise avec les deux lames 160, 170.

Comme indiqué précédemment, en position de fermeture des lames 160, 170, le dissecteur peut être placé à l'intérieur de la canule 200 comme représenté sur les figures 1 et 2. Pour utiliser le dissecteur il suffit, après avoir placé l'extrémité proximale 204 de la canule 200 au voisinage des tissus ou organes sur lesquels on souhaite intervenir, de déplacer le dissecteur 100 à translation par rapport à la canule 200, selon l'axe 206 de sorte que les lames 160, 170 soient placées à l'extérieur de la canule 200 comme représenté sur les figures 3 et 4 ; puis de tirer sur l'élément de commande 120 dans le sens distal, pour déplacer le doigt 125 sur les rampes 162, 172 et provoquer l'ouverture des lames 160, 170 comme représenté sur les figures 3 et 4, accentuant et facilitant ainsi le clivage entre les tissus. Ceci peut par exemple se faire d'un geste naturel de la main, en regroupant l'index vers le pouce, respectivement engagés dans les oeillets 123, 113.

Les lames 160, 170 reprennent la position de repos fermée dès que la traction sur l'élément de commande 120 est relachée.

La platine 110 et l'élément de commande 120 peuvent bien entendu présenter une certaine souplesse transversale afin de suivre les déformations ou ondulations éventuelles de la canule 200 pour atteindre le lieu d'intervention, sous réserve que la platine 110 et l'élément de commande soient rigides à la traction.

Les lames 160, 170 présentent des bords extérieurs 164, 174 effilés non tranchants adaptés au clivage. Dans ce cas le dissecteur 100 peut être utilisé en position fermée des lames 160, 170. Par ailleurs, la manoeuvre à l'ouverture et à la fermeture des lames 160, 170 facilite le clivage des tissus. Les lames 160 et 170 ont leur partie proximale avantageusement incurvée au-dessus du plan contenant l'axe 206 de la canule et perpendiculaire au plan axial 140, comme on le remarque à l'examen de la figure 1.

On peut concevoir également de réaliser les lames 160 et 170 dans un matériau à mémoire de forme, c'est-à-dire à grande élasticité, ce qui permettrait d'incurver davantage encore les lames 160 et 170, tout en conservant la possibilité de rentrer l'ensemble dans la canule 200.

Ainsi, dans une variante de réalisation avantageuse de l'invention, les lames 160 et 170 sont réalisées dans un matériau à mémoire de forme et configurées dans une position de repos où elles sont très largement incurvées, typiquement jusqu'à former un angle droit, au-dessus du plan contenant l'axe 206 de la canule et perpendiculaire au plan axial 140, l'extrémité proximale des lames faisant saillie au-dessus du plan tangent supérieur à la canule 200 perpendiculaire au plan axial 140.

Les lames peuvent alors rentrer en se dépliant de la forme incurvée vers une forme plus plane dans la canule par simple traction sur la platine 120. On procède de même en dépliant élastiquement les lames lors de l'introduction de la platine support dans la canule.

La géométrie incurvée des lames selon cette variante de réalisation permet de cliver les tissus entourant par exemple un vaisseau sanguin.

Dans une variante de réalisation, on peut rendre tranchant les bords internes 163, 173 des lames 160, 170.

Selon une variante de réalisation le doigt de commande 125 cylindrique de révolution autour de l'axe 126 selon la représentation des figures 1 à 4 peut être remplacé par un coin adapté à la géométrie des rampes 162, 172.

Bien entendu la présente invention n'est pas limitée au mode de réalisation particulier qui vient d'être décrit mais s'étend à toutes variantes conformes à son esprit.

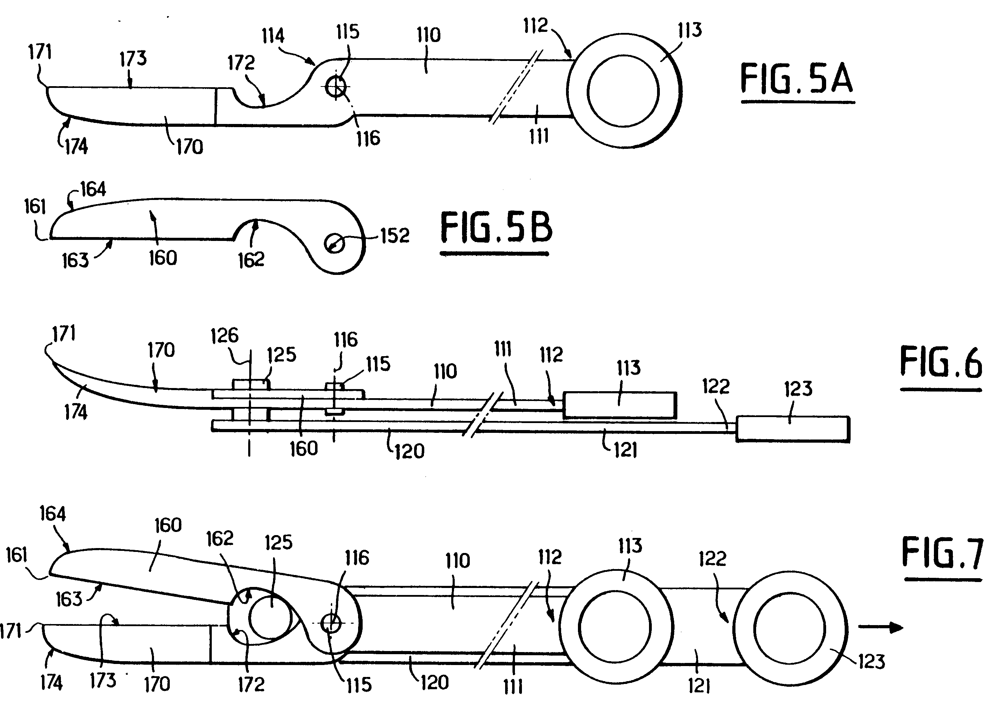

Ainsi par exemple, si dans le mode de réalisation précédemment décrit en regard des figures 1 à 4, les deux lames 160, 170 sont formées d'une pièce unique repliée sur elle-même, comme représenté sur les figures 5A à 10C, les deux lames 160, 170 peuvent être formées de pièces distinctes.

Plus précisément, selon le mode de réalisation représenté sur les figures 5A à 7, les deux lames 160, 170 sont séparées l'une de l'autre, mais l'une des lames, en l'espèce la lame 170, est liée rigidement à l'extrémité proximale 114 de la platine 110. Pour le reste, le dissecteur 100 reste pratiquement identique à celui décrit précédemment en regard des figures 1 à 4. La seconde lame 160 est montée à pivotement sur la platine 110, par rapport à la lame 170 autour du tourillon 115 porté à l'extrémité proximale 114 de la platine 110. Par ailleurs, l'élément de commande 120 est muni à son extrémité proximale d'un doigt de commande 125 qui coopère avec des rampes 162, 172 prévues respectivement sur chacune des lames 160, 170.

On notera cependant que lorsque les lames 160, 170 sont réalisées sous forme de pièces séparées, il est nécessaire de prévoir un élément élastique sollicitant les lames 160, 170 vers une position de repos. On préfèrera la position de repos correspondant à la position de fermeture selon la représentation donnée sur les figures 5A à 7.

Le fonctionnement du dissecteur représenté sur les figures 5A à 7 reste identique à celui du premier mode de réalisation représenté sur les figures 1 à 4, à ceci près que lors de la traction de l'élément commande 120, seule la lame 160 est déplacée à pivotement par rapport à la platine 110.

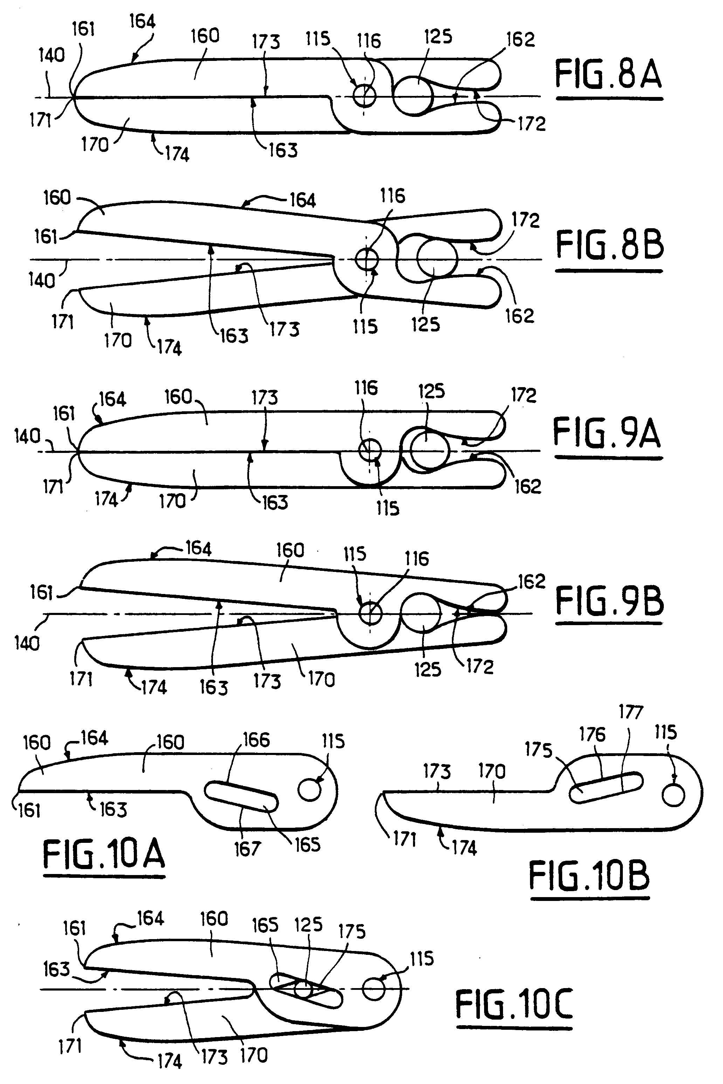

On retrouve sur les figures 8A et 8B deux lames 160, 170 distinctes articulées sur le tourillon 115 porté par la platine 110. Le mode de réalisation représenté sur les figures 8A et 8B se distinguent de ceux précédemment décrits et représentés sur les figures 1 à 7 par le fait que selon la représentation des figures 8A et 8B, les rampes 162, 172 sont prévues sur le côté distal du tourillon 115. On notera que pour maintenir une sollicitation à la fermeture des lames 160, 170 lors d'une traction sur l'élément de commande 120 dans le sens distal, il est nécessaire d'inverser, par rapport au plan de symétrie 140, les positions respectives des rampes 162, 172.

Ainsi, si la lame 160 est placée sur un premier côté du plan de symétrie 140, la rampe 162 associée est placée sur le côté opposé. Il en est de même pour la seconde lame 170.

Les différents modes de réalisation précédemment décrits et représentés sur les figures 1 à 8B comprennent des lames 160, 170 sollicitées à l'ouverture par traction sur l'élément de commande 120 dans le sens distal. En variante, comme représenté sur les figures 9A et 9B, les lames 160, 170 peuvent être sollicitées à la fermeture par traction sur l'élément de commande 120 dans le sens distal.

On aperçoit sur les figures 9A et 9B deux lames distinctes 160, 170 articulées autour du tourillon 115 porté par la platine 110. Les rampes 162, 172 sont prévues respectivement sur chaque lame 160, 170, sur le côté distal du tourillon 115. Les rampes 162, 172 sont prévues respectivement du même côté que les lames 160, 170 par rapport au plan de symétrie 140. Les rampes 162, 172 convergent en éloignement du tourillon 115.

Le doigt de commande 125 prévu sur l'élément de commande 120 est placé entre les rampes 162, 172.

Selon les différents modes de réalisation précédemment décrits et représentés sur les figures 1 à 9B, les lames 160, 170 sont sollicitées vers une position de repos (la position fermée selon les figures 1 à 8B et la position ouverte selon les figures 9A, 9B) par un organe élastique. Comme indiqué précédemment, cet organe élastique peut être formé par la pièce 150 composant les lames 160, 170 lorsque celles-ci sont formées d'une pièce unique, ou encore d'un ressort séparé.

Toutefois de tels moyens élastiques peuvent être supprimés lorsque les lames 160, 170 comportent une double rampe coopérant avec le doigt 125 pour solliciter alternativement les lames vers la position de fermeture et vers la position d'ouverture.

Cette double rampe peut être formée comme représenté sur les figures 10A à 10C d'une lumière 165, 175. Les lumières 165, 175 ont leurs bords 166, 167 et 176, 177 parallèles. Ces bords 166, 167 et 176, 177 sont inclinés par rapport à la direction de translation de l'élément de commande 120 qui coïncide avec le plan de symétrie 140. Les lumières 165, 175 reçoivent le doigt de commande 125 porté par l'élément de commande 120, doigt de commande 125 dont le diamètre correspond à l'écart entre les paires de surfaces 166, 167 d'une part, et 176, 177 d'autre part.

Les lames 160, 170 peuvent être rigoureusement symétriques par rapport au plan 140 précité.

L'homme de l'art comprendra aisément que le déplacement du doigt de commande 125 selon le plan de symétrie 140, permet de solliciter les lames 160, 170 alternativement à la fermeture et à l'ouverture.

On rappelle que l'utilisation d'une double rampe comme représenté sur les figures 10A et 10C permet de s'affranchir de l'organe élastique.

Bien entendu la platine 110 et l'élément de commande 120 peuvent faire l'objet de nombreuses variantes de réalisation. A titre d'exemple, on peut réaliser la platine 110 et l'élément de commande 120 sous forme de tubes concentriques. L'élément de commande 120, représenté schématiquement sous forme d'une tige rigide sur les figures annexées, peut être remplacé par un câble lorsqu'il opère par traction.

Selon encore une autre variante, on peut envisager de placer deux doigts de commande 125 sur l'extrémité proximale 124 de l'élément de commande 120, à la façon d'une fourche, coopérant respectivement avec les deux lames 160, 170.

**Revendications**

1. Dissecteur, notamment pour chirurgie endoscopique, caractérisé par le fait qu'il comprend :
   – une canule (200),
   – une platine support (110) placée dans la canule (200) et formée d'une tringle allongée (111) de longueur supérieure à celle de la canule (200),
   – un élément de commande (120) placé également dans la canule, susceptible de translation par rapport à la platine support (110) et pourvu d'un doigt de commande (115) transversal à sa direction de translation, et
   – deux lames (160, 170) portées par la platine (110), dont l'une au moins est articulée sur la platine (110) et possède une rampe (162, 172) inclinée par rapport à la direction de translation de l'élément de commande (120) et sur laquelle repose le doigt de commande (125), de sorte que le déplacement à translation de l'élément de commande (120), par rapport à la platine support (110), entraîne un pivotement relatif des deux lames (160, 170) dont le bord extérieur non tranchant est adapté au clivage.

2. Dissecteur selon la revendication 1, caractérisé par le fait que les deux lames (160, 170) sont formées d'une pièce unique (150).

3. Dissecteur selon la revendication 1, caractérisé par le fait que les deux lames (160, 170) sont formées de pièces séparées.

4. Dissecteur selon l'une des revendications 1 ou 3, caractérisé par le fait que l'une des lames (160, 170) est solidaire de la platine (110).

5. Dissecteur selon l'une des revendications 1 à 4, caractérisé par le fait que les deux lames (160, 170) sont sollicitées élastiquement vers une position de repos.

6. Dissecteur selon la revendication 5, caractérisé par le fait que la position de repos correspond à la position fermée des lames (160, 170).

7. Dissecteur selon la revendication 5, caractérisé par le fait que la position de repos correspond à la position ouverte des lames (160, 170).

8. Dissecteur selon l'une des revendications 5 à 7, caractérisé par le fait que les lames (160, 170) sont formées d'une pièce unique (150) possédant une élasticité inhérente apte à solliciter les lames (160, 170) vers une position de repos.

9. Dissecteur selon l'une des revendications 1 à 8, caractérisé par le fait que les lames (160, 170) possèdent un bord extérieur arrondi non tranchant (164, 174).

10. Dissecteur selon l'une des revendications 1 à 9, caractérisé par le fait que les lames (160, 170) possèdent un bord intérieur tranchant (163, 173).

FIG.1

FIG.2

EP 0 485 279 A1

FIG.3

FIG.4

EP 0 485 279 A1

EP 0 485 279 A1

**FIG.5A**

**FIG.5B**

**FIG.6**

**FIG.7**

FIG.8A

FIG.8B

FIG.9A

FIG.9B

FIG.10A

FIG.10B

FIG.10C

EP 0 485 279 A1

Office européen
des brevets

**RAPPORT DE RECHERCHE EUROPEENNE**

Numero de la demande

EP    91 40 2966

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 380 874 (BARD)<br>* colonne 7, ligne 16 - ligne 24 *<br>* colonne 8, ligne 47 - colonne 9, ligne 48; figures 4,6,6A,6B *<br>--- | 1-10 | A61B17/32 |
| X | DE-U-8 900 376 (STORZ)<br>* page 8, ligne 4 - page 11, ligne 1; figures 1-6 *<br>--- | 1,4 | |
| A | EP-A-0 276 104 (AMERICAN MEDICAL SYSTEMS)<br>* abrégé; figures 2A,B *<br>--- | 1,5,6 | |
| A | EP-A-0 279 957 (BEAVER)<br>* colonne 3, ligne 25 - ligne 42; figure 1 *<br>----- | 1 | |

|  |  | **DOMAINES TECHNIQUES RECHERCHES (Int. Cl.5)** |
|---|---|---|
|  |  | A61B |

Le présent rapport a été établi pour toutes les revendications

| Lieu de la recherche | Date d'achèvement de la recherche | Examinateur |
|---|---|---|
| LA HAYE | 10 FEVRIER 1992 | MOERS R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P0402)

10